# EUROPÄISCHE PATENTSCHRIFT

(11) **EP 3 797 697 B1**
(45) Veröffentlichungstag und Bekanntmachung des Hinweises auf die Patenterteilung: **27.03.2024**
(21) Anmeldenummer: 19200206.1
(22) Anmeldetag: 27.09.2019
(51) Int. Cl.: A61B 6/02, A61B 6/00, G06T 5/50, G06T 7/30

(54) **TOMOSYNTHESEVERFAHREN MIT KOMBINIERTEN SCHICHTBILDDATENSÄTZEN**
TOMOSYNTHESIS METHOD WITH COMBINED LAYER IMAGE DATA SETS
PROCÉDÉ DE TOMOSYNTHÈSE COMPRENANT DES ENSEMBLES DE DONNÉES D'IMAGE DE COUCHE COMBINÉS

(43) Veröffentlichungstag der Anmeldung: 31.03.2021
(73) Patentinhaber: Siemens Healthineers AG, 91301 Forchheim (DE)
(72) Erfinder: Ritschl, Ludwig, 91054 Erlangen (DE)
(74) Vertreter: Siemens Healthineers Patent Attorneys

(56) Entgegenhaltungen:
- US-A1- 2014 254 910
- US-A1- 2016 166 329
- US-A1- 2016 334 964
- US-A1- 2017 132 792
- US-A1- 2019 117 180
- US-B1- 9 741 127

## Beschreibung

Die Erfindung betrifft ein Verfahren zur Aufnahme von mindestens zwei um einen vorbestimmten Untersuchungswinkel versetzte Tomosyntheseaufnahmen eines Untersuchungsobjekts, welche zu einer gemeinsamen Anzeige kombiniert werden können.

Üblicherweise werden Tomosyntheseverfahren bei der Brustbildgebung verwendet. Die Röntgenquelle kann dabei parallel zur Kompressionsvorrichtung bzw. dem Röntgendetektor linear verschiebbar angeordnet sein. Alternativ oder zusätzlich kann der Röntgenstrahler auf einem Kreisbogen verschiebbar angeordnet sein, wobei der Kreisbogen um eine Drehachse definiert ist, welche senkrecht zur Systemachse eines Mammographiesystems ausgerichtet ist. Damit kann eine dreidimensionale Röntgenbildgebung, insbesondere eine Tomosynthese, mittels der Mammographieanlage durchgeführt werden. Im Allgemeinen kann bei einer Tomosynthese ein dreidimensionales Bild aus zweidimensionalen Bildern, welche aus verschiedenen Winkeln der Röntgenquelle relativ zum Röntgendetektor erfasst werden, erzeugt werden. Die zweidimensionalen und/oder dreidimensionalen Bilder können Teil eines Tomosynthese-(Bild-)Datensatzes sein.

Zunehmend werden Tomosyntheseverfahren auch für orthopädische Anwendungen diskutiert. Aus der Veröffentlichung C. Luckner et al, "Parallel-Shift Tomosynthesis for Orthopedic Applications", SPIE Medical Imaging, Houston, 2018 ist beispielsweise ein Tomosyntheseverfahren für orthopädische Fragestellungen bekannt.

Aus der Druckschrift US 2014 / 254910 A1 ist ein Verfahren zur Zuordnung von ersten Lokalisierungsdaten einer Brust eines Patienten bekannt, wobei die ersten Lokalisierungdaten aus ersten Bilddaten der Brust abgeleitet werden, wobei die ersten Bilddaten das Ergebnis eines ersten radiologischen Datenerfassungsprozesses sind, zu zweiten Lokalisierungsdaten derselben Brust, die aus zweiten Bilddaten abgeleitet werden, wobei die zweiten Bilddaten das Ergebnis eines zweiten radiologischen Datenerfassungsprozesses sind, oder umgekehrt.

Aus der Druckschrift US 2016 / 166329 A1 ist eine Identifizierung und Verfolgung eines Navigationsinstruments (z. B. einer Nadel) in drei Dimensionen, mit Bildaktualisierungen des Instruments im Wesentlichen in Echtzeit und Aktualisierungen des Gewebes mit gleicher oder niedrigerer Rate bekannt.

Aus der Druckschrift US 2016 / 334964 A1 ist eine Benutzerschnittstelle bekannt, die eine markierungsbasierte Interaktion für Bilder ermöglicht. Die Benutzerschnittstelle ermöglicht die eine markierungsbasierte Interaktion für Bilder, wobei die Bilder ein Volumen, das ein dreidimensionales Bild ist, und Scheiben, die zweidimensionale Bilder sind, umfassen, von denen jedes einen Querschnitt des Volumens darstellt.

Aus der Druckschrift US 2017 / 132792 A1 ist ein Verfahren bekannt, welches einen diagnostizierenden Arzt bei der Beschreibung der Lage einer Zielstruktur in einem Tomosynthese-Bilddatensatz einer komprimierten Brust eines Patienten unterstützt. Die Zielstruktur wird durch ein erstes räumliches Informationselement lokalisiert.

Aus der Druckschrift US 9 741 127 B1 ist eine Bildverarbeitungsvorrichtung bekannt, welche ein Operationsbild durch Addieren oder Subtrahieren eines Originalbildes und eines Standardabweichungsbildes erzeugt, wobei das Standardabweichungsbild die Standardabweichung für die Pixel abbildet, die das Originalbild konfigurieren. In diesem Operationsbild werden die Bilder der Strukturen, die in Teilen des Originalbildes zu sehen sind, die keine Metallteile sind, gelöscht. Folglich erscheinen Strukturen, bei denen es sich nicht um Metallteile handelt, die im Originalbild z. B. in einer weißlichen Farbe erscheinen, nicht in dem Operationsbild. Wenn ein solches Operationsbild einer Binarisierungsverarbeitung unterzogen wird, bei der die Metallteile, die beispielsweise in einer weißlichen Farbe erscheinen, extrahiert werden, da dann eine genaue Graphenschnittverarbeitung möglich ist, erscheinen Bilder, die von Strukturen stammen, die keine Metallteile sind, nicht im resultierenden Bild.

Aus der Druckschrift US 2019 / 0 117 180 A1 ist ein Verfahren zur Bestimmung einer Ausrichtung zwischen mindestens zwei Knochenteilen eines langgestreckten Knochensystems eines Patienten bekannt, welches die Aufzeichnung einer Vielzahl von sich teilweise räumlich überlappenden Projektionsbildern durch ein Aufzeichnungssystem eines Röntgengeräts während einer Translationsbewegung des Röntgengeräts oder des Aufzeichnungssystems in einer Richtung von oder parallel zu einer Längsachse des Knochensystems umfasst. Aus den aufgezeichneten Projektionsbildern werden Tomosynthese-Bilddaten der Knochenteile rekonstruiert, und ein Ausrichtungswinkel zwischen den mindestens zwei Knochenteilen wird zumindest teilweise auf der Grundlage der rekonstruierten Tomosynthese-Bilddaten und/oder der Vielzahl von Projektionsbildern bestimmt oder geschätzt.

Eine gleichzeitige Darstellung beispielsweise koronaler und sagittaler Schnittbilder eines Untersuchungsobjekts beruht typischerweise auf einer vollständigen dreidimensionale Rekonstruktion. Der Erfinder hat das Problem erkannt, dass dazu einerseits die Möglichkeit einer Computertomographieaufnahme sowie andererseits eine Mindestdosis für die Computertomographieaufnahme nötig ist. Insbesondere bei orthopädischen Fragestellungen wird eine Aufnahme unter Belastung der Gelenke, d.h. im Stehen, bevorzugt. Eine Computertomographieaufnahme eines stehenden Patienten ist nicht in allen Kliniken oder Praxen möglich.

Es ist Aufgabe der Erfindung, ein Verfahren zur Aufnahme von mindestens zwei um einen vorbestimmten Untersuchungswinkel versetzte Tomosyntheseaufnahmen eines Untersuchungsobjekts, eine Bilderzeugungseinheit, ein medizinischen Röntgensystem, ein Computerprogrammprodukt und ein computerlesbares Medium anzugeben, welche eine gleichzeitige Darstellung von Schichtbildern, welche aus unterschiedlichen Blickrichtungen aufgenommen wurden, mit einer verringerten Dosis ermöglichen.

Die Aufgabe wird erfindungsgemäß gelöst durch ein Verfahren zur Aufnahme von mindestens zwei um einen vorbestimmten Untersuchungswinkel versetzte Tomosyntheseaufnahmen eines Untersuchungsobjekts nach Anspruch 1, eine Bilderzeugungseinheit nach Anspruch 11, ein medizinischen Röntgensystem nach Anspruch 12, ein Computerprogrammprodukt nach Anspruch 13 und ein computerlesbares Medium nach Anspruch 14.

Die Erfindung betrifft ein Verfahren zur Aufnahme von mindestens zwei um einen vorbestimmten Untersuchungswinkel versetzte Tomosyntheseaufnahmen eines Untersuchungsobjekts. Das Untersuchungsobjekt ist zwischen einer Röntgenquelle und einem Röntgendetektor angeordnet. Die Röntgenquelle und/oder der Röntgendetektor werden in gegenüberliegenden parallelen Ebenen, insbesondere parallel gegenüberliegend, entlang einer linearen Trajektorie bewegt. Das erfindungsgemäße Verfahren weist die Schritte des ersten Aufnehmens, des zweiten Aufnehmens, des Ermittelns und des (Bild-)Registrierens auf. Im Schritt des ersten Aufnehmens wird eine Mehrzahl von ersten Projektionsaufnahmen entlang der linearen Trajektorie aufgenommen, wobei die Röntgenquelle und der Röntgendetektor das Untersuchungsobjekt in einer ersten Aufnahmeebene aufnehmen. Im Schritt des zweiten Aufnehmens wird eine Mehrzahl von zweiten Projektionsaufnahmen entlang der linearen Trajektorie aufgenommen, wobei die Röntgenquelle und der Röntgendetektor das Untersuchungsobjekt in einer von der ersten verschiedenen zweiten Aufnahmeebene aufnehmen, wobei die erste Aufnahmeebene und die zweite Aufnahmeebene den Untersuchungswinkel einschließen. Im Schritt des zweiten Aufnehmens können sich die Röntgenquelle und der Röntgendetektor in der gleichen Richtung oder in der entgegengesetzten Richtung im Vergleich zum ersten Aufnehmen entlang der linearen Trajektorie bewegen. Im Schritt des Ermittelns wird ein erster Schichtbilddatensatz basierend auf den ersten Projektionsaufnahmen und von einem zweiten Schichtbilddatensatz basierend auf den zweiten Projektionsaufnahmen ermittelt. Im Schritt des Registrierens wird der erste Schichtbilddatensatz und der zweite Schichtbilddatensatz, insbesondere aufeinander, registriert.

Der Erfinder hat erkannt, dass anstatt einer vollständigen dreidimensionalen Rekonstruktion bzw. einer Computertomographieaufnahme zwei, beispielsweise um 90 Grad, versetzte Tomosyntheseaufnahmen erzeugt werden können, die jeweils in einem Untersuchungswinkel, beispielsweise orthogonal, zueinanderstehende Schnitt- bzw. Schichtbilddatensätze ermöglichen. Vorteilhaft kann die Untersuchung mit deutlich reduzierter Dosis durchgeführt werden. Vorteilhaft kann die Aufnahme innerhalb einer kurzen Zeitdauer durchgeführt werden. Vorteilhaft kann im Vergleich zu einer Computertomographieaufnahme eine verbesserte Genauigkeit für hohe Ortsfrequenzen erreicht werden.

Die Körperlängsachse des Untersuchungsobjekts bzw. eine Längsachse der Untersuchungsregion des Untersuchungsobjekts, beispielsweise eine Extremität, wird im Wesentlichen parallel zur linearen Trajektorie ausgerichtet. Die lineare Trajektorie kann beispielsweise entlang der Körperlängsachse durch den Körpermittelpunkt verlaufen.

Der Röntgendetektor und/oder die Röntgenquelle bewegen sich entlang der linearen Trajektorie innerhalb einer Ebene, dabei spannt sich eine Aufnahmeebene auf. Entlang der linearen Trajektorie kann bedeuten, dass sich der Röntgendetektor als auch die Röntgenquelle in die gleiche Richtung entlang der linearen Trajektorie bewegen oder dass sich zumindest die Röntgenquelle oder der Röntgendetektor in Richtung der linearen Trajektorie bewegen oder dass sich der Röntgendetektor und die Röntgenquelle in gegenläufigen Richtungen entlang bzw. parallel zur linearen Trajektorie bewegen. Alle während des ersten Aufnehmens ausgelesenen Messwerte des Röntgendetektors werden in der ersten Aufnahmeebene gemessen. Alle während des zweiten Aufnehmens ausgelesenen Messwerte des Röntgendetektors werden in der zweiten Aufnahmeebene gemessen.

Aus den gemessenen Werten des Röntgendetektors, welche den Projektionsdatensatz bilden kann in einem Schritt des Rekonstruierens ein Tomosynthesedatensatz erzeugt werden, wobei die Tiefeninformation des Untersuchungsobjekts jeweils entlang von Röntgenstrahlen des Röntgenstrahlenbündels der Röntgenquelle ermittelt wird, so dass unterschiedliche Tiefenebenen im Objekt parallel zur Detektionsfläche des Röntgendetektors eine unterschiedliche Abtastung aufweisen. Das Röntgenstrahlenbündel kann bei einer gleichzeitigen und parallelen Bewegung vom Röntgendetektor und der Röntgenquelle in dieselbe Richtung entlang der linearen Trajektorie insbesondere durch die Bewegung entlang der linearen Trajektorie und dem Röntgenstrahlenfächer der Röntgenquelle senkrecht zur linearen Trajektorie aufgespannt werden, d.h. das Röntgenstrahlenbündel kann dachförmig sein. Für andere Bewegungsformen von der Röntgenquelle und dem Röntgendetektor entlang der linearen Trajektorie, beispielsweise stehende Röntgenquelle oder gegenläufige Bewegung in entgegengesetzter Richtung, kann ein im Wesentlichen kegelförmiges Röntgenstrahlenbündel ausgebildet sein. Das Rekonstruieren des Tomosynthesedatensatzes kann mittels einer Rückprojektion oder Maximum-Likelihood-Methode durchgeführt werden. Durch die Rekonstruktion können ein Schnitt- bzw. Schichtbild bzw. eine Tiefeninformation ermittelt werden. Der Tomosynthesedatensatz kann insbesondere ein zumindest teilweiser Volumendatensatz sein.

In einem Schritt des Ermittelns kann ein Schichtbild mit einer Schichtdicke in einer im Wesentlichen zur Detektionsfläche des Röntgendetektors parallelen Tiefenebene basierend auf dem Tomosynthesedatensatz ermittelt werden. Das Schichtbild wird in x-z-Richtung bzw. α-z-Richtung ermittelt. Das Schichtbild kann insbesondere mittels Vorwärtsprojektion basierend auf dem Tomosynthesedatensatz ermittelt werden. Die Schichtdicke kann entlang der y-Richtung bestimmt werden. Das Schichtbild, insbesondere die Schichtmitte, ist einem y-Wert zugeordnet.

Im Schritt des Registrierens werden der erste Schichtbilddatensatz und der zweite Schichtbilddatensatz aufeinander registriert. Das Registrieren kann auch als Bildregistrieren bezeichnet werden. Der erste Schichtbilddatensatz und der zweite Schichtbilddatensatz können basierend auf bekannten und an die Geometrie hinsichtlich der x-z-Richtung bzw. α-z-Richtung angepassten Methoden der Bildregistrierung erfolgen. Dazu können Positionsdaten bei der Aufnahme, beispielsweise die Position der Röntgenquelle und/oder des Röntgendetektors, genutzt werden. Die Darstellung der Schichtbilder kann im kartesischen Koordinatensystem (x,y,z) erfolgen. Alternativ kann die Darstellung der Schichtbilder in einem speziellen Koordinatensystem (α,y,z) erfolgen, welches den Pfadwinkel α berücksichtigt.

Gemäß einem Aspekt der Erfindung wird beim Ermitteln des ersten Schichtbilddatensatzes und des zweiten Schichtbilddatendatensatzes die Tiefeninformation jeweils entlang eines Pfades mit einem Pfadwinkel gegenüber einer kartesischen Raumrichtung ermittelt.

Die unterschiedlichen Tiefenebenen im Objekt parallel zur Detektionsfläche weisen eine unterschiedliche Abtastung auf. Dabei weist eine Tiefenebene, welche näher an der Röntgenquelle angeordnet ist eine höhere räumliche Abtastung auf als eine davon verschiedene Tiefenebene, welche von der Röntgenquelle weiter entfernt angeordnet ist. Die räumliche Abtastung in x-Richtung bzw. der x-z-Ebene ist damit abhängig von der y-Position der Tiefenebene bzw. eines Datenpunkts des Tomosynthesedatensatzes.

Gemäß einem Aspekt der Erfindung wird im Schritt des Registrierens jeweils der Pfadwinkel im ersten Schichtbilddatensatz und im zweiten Schichtbilddatensatz berücksichtigt. Der Pfadwinkel beträgt α=sin(x/SID), wobei die x-Richtung die kartesische Raumrichtung ist und SID dem Abstand zwischen der Röntgenquelle und dem Röntgendetektor in y-Richtung entspricht. Vorteilhaft kann eine verbesserte Tiefeninformation gegenüber einer einfachen, projektiven Radiographieaufnahme über das Objekt ermittelt werden. Vorteilhaft kann diese winkelabhängige Tiefeninformation bei der Berechnung von Schichtbildern verwendet werden, um insbesondere bei größer werdenden Pfadwinkeln eine korrektere Berechnung von insbesondere dicken Schichtbildern zu ermöglichen.

Gemäß einem Aspekt der Erfindung ist der Untersuchungswinkel untersuchungsabhängig. Die erste und zweite Aufnahmeebene kann können in Abhängigkeit der Untersuchung, der Untersuchungsregion oder der Untersuchungsart gewählt werden. Vorteilhaft können genau die Aufnahmeebenen aufgenommen werden, welche zur Diagnose benötigt werden.

Gemäß einem Aspekt der Erfindung beträgt der Untersuchungswinkel im Wesentlichen 90 Grad. Es können beispielsweise eine koronale und sagittale Aufnahmeebene gewählt werden.

Gemäß einem Aspekt der Erfindung wird basierend auf dem ersten Schichtbilddatensatz und dem zweiten Schichtbilddatensatz ein Abstand von anatomischen Merkmalen oder Landmarken bestimmt. Es können insbesondere dreidimensionale Messungen von Knochenstellungen durchgeführt werden. Dazu können anatomische Merkmale oder Landmarken im ersten und zweiten Schichtbilddatensatz markiert werden. Das Markieren kann automatisch mittels einer automatischen Erkennung, beispielsweise basierend auf einer Segmentierung oder einer Bilderkennung, erfolgen. Alternativ oder zusätzlich kann das Markieren durch einen Benutzer erfolgen. Der Abstand zwischen den anatomischen Merkmalen bzw. Landmarken, wobei mindestens eine im ersten Schichtbilddatensatz und mindestens eine andere im zweiten Schichtbilddatensatz markiert ist, kann bestimmt werden, wobei jeweils der Pfadwinkel im ersten Schichtbilddatensatz und im zweiten Schichtbilddatensatz berücksichtigt wird. Vorteilhaft kann eine verbesserte Messung von Knochenstellungen basierend auf Aufnahmen mit einem Radiographiesystem ermöglicht werden. Vorteilhaft kann die Patientendosis reduziert werden, da zur Messung statt Computertomographieaufnahmen Aufnahmen des Radiographiesystems genutzt werden können. Der Begriff dreidimensionale Messung bedeutet insbesondere, dass die anatomischen Merkmale bzw. Landmarken nicht innerhalb eines einzigen Schichtbilds vorhanden sind und nur dort markiert werden. Die anatomischen Merkmale bzw. Landmarken können insbesondere in mehreren Schichtbildern im ersten und/oder zweiten Schichtbilddatensatz verteilt sein und im jeweiligen Schichtbild markiert werden. Im Wesentlichen exakte 3D-Punkte im Objekt können durch die kombinierte Auswahl zweier Landmarken im ersten Schichtbilddatensatz und im zweiten Schichtbilddatensatz bestimmt werden. Dies ermöglicht vorteilhaft dreidimensionale Messungen von Knochenstellungen sowie die Planung von Implantaten innerhalb einer dreidimensionalen Darstellung.

Gemäß einem Aspekt der Erfindung werden der erste Schichtbilddatensatz und der zweite Schichtbilddatensatz gemeinsam angezeigt. Der erste Schichtbilddatensatz und der zweite Schichtbilddatensatz können insbesondere gleichzeitig angezeigt werden. Der erste Schichtbilddatensatz und der zweite Schichtbilddatensatz können insbesondere nebeneinander angezeigt werden. Vorteilhaft können bei der Diagnose Informationen aus verschiedenen Aufnahmeebenen bzw. Perspektiven genutzt werden.

Gemäß einem Aspekt der Erfindung wird mittels einer Benutzereingabe in einem ersten Schichtbild des ersten Schichtbilddatensatzes ein korrespondierendes zweites Schichtbild im zweiten Schichtbilddatensatz angezeigt. Der Benutzer kann mittels einer Eingabe, zum Beispiel durch Anklicken einer Position in einem Schichtbild des ersten oder zweiten Schichtbilddatensatzes, die Anzeige des anderen Schichtbilddatensatzes beeinflussen, so dass beispielsweise die Position in beiden Schichtbilddatensätzen in dem die Position jeweils enthaltenen Schichtbild dargestellt wird. Bei der Bestimmung der Position im anderen Schichtbilddatensatz wird jeweils der Pfadwinkel im ersten Schichtbilddatensatz und im zweiten Schichtbilddatensatz berücksichtigt. Vorteilhaft kann eine verbesserte Transformation einer Position von einem Schichtbilddatensatz in den anderen Schichtbilddatensatz durchgeführt werden.

Gemäß einem Aspekt der Erfindung wird mittels der Benutzereingabe ein Schieberegler in einem ersten Schichtbild des ersten Schichtbilddatensatzes bewegt, so dass das der Tiefe des momentan angezeigten Schiebereglers entsprechende zweite Schichtbild des zweiten Schichtbilddatensatzes angezeigt wird. Durch Betätigen bzw. Verschieben des Schiebereglers in einem ersten Schichtbild des ersten Schichtbilddatensatzes kann das entsprechend anzuzeigende zweite Schichtbild des zweiten Schichtbilddatensatzes ausgewählt werden. Die Anzeige des ersten und zweiten Schichtbilddatensatzes kann damit synchronisiert werden. Wird beispielsweise ein vertikaler Balken im ersten Schichtbild an einem x- bzw. α-Wert angezeigt, so wird als zweites Schichtbild dasjenige Schichtbild angezeigt, wobei die Tiefe bzw. der y-Wert des zweiten Schichtbildes dem x- bzw. α-Wert im ersten Schichtbild im Wesentlichen entspricht. Das erste Schichtbild, beispielsweise eine Umrandung entlang der Außenkanten des ersten Schichtbilds, und der Schieberegler im zweiten Schichtbild können farblich identisch kodiert werden. Die Tiefe bzw. der y-Wert des ersten Schichtbildes kann entsprechend mittels eines Schiebereglers an einem entsprechenden x- bzw. α-Wert im zweiten Schichtbild eingeblendet bzw. angezeigt werden. Das zweite Schichtbild, beispielsweise eine Umrandung entlang der Außenkanten des zweiten Schichtbilds, und der Schieberegler im ersten Schichtbild können farblich identisch kodiert werden, wobei sich die Farbe von der Kodierung des ersten Schichtbilds und des Schiebereglers im zweiten Schichtbild unterscheidet. Beispielsweise können zwei verschieden Grundfarben bzw. komplementär Farben verwendet werden.

Der Schieberegler kann beispielsweise in Form eines Balkens entlang der x- oder z-Achse bzw. α- oder z-Achse in einem Schichtbild der ersten oder zweiten Schichtbilddatensatzes eingeblendet werden. Der Schieberegler kann als horizontaler oder vertikaler Balken in einem Schichtbild der ersten oder zweiten Schichtbilddatensatzes eingeblendet werden.

Der zweite Schichtbilddatensatz kann mittels Bewegens des Schiebereglers bzw. Balkens, der im ersten Schichtbilddatensatz eingeblendet bzw. überlagert angezeigt wird, durchscrollt werden. Gleiches ist auch anders herum möglich, also dass der erste Schichtbilddatensatz mittels Bewegens des Schiebereglers bzw. Balkens, der im zweiten Schichtbilddatensatz eingeblendet bzw. überlagert angezeigt wird, durchscrollt werden kann.

Gemäß einem Aspekt der Erfindung wird der Verlauf einer sich erstreckenden anatomischen Struktur in einem ersten Schichtbild des ersten Schichtbilddatensatzes markiert und ein zusammengesetztes Schichtbild basierend auf dem markierten Verlauf und basierend auf dem zweiten Schichtbilddatensatz ermittelt. Das zusammengesetzte Schichtbild kann als zweites Schichtbild angesehen werden. Das zusammengesetzte Schichtbild kann angezeigt werden, beispielsweise als zweites Schichtbild oder zusätzlich zum zweiten Schichtbild. Die Markierung kann beispielsweise durch den Benutzer mittels des Cursors erfolgen. Die Markierung kann mittels Segmentierung oder automatischer Bilderkennung erfolgen. Durch Auswahl bzw. Markierung einer insbesondere gekrümmten Linie in einem ersten Schichtbild des ersten Schichtbilddatensatzes kann eine sogenannte "curved MPR", d.h. eine gekrümmte multiplanare Reformation, basierend auf dem zweiten Schichtbilddatensatz erzeugt werden. Alternativ kann durch Auswahl bzw. Markierung einer insbesondere gekrümmten Linie in einem zweiten Schichtbild des zweiten Schichtbilddatensatzes eine sogenannte "curved MPR" basierend auf dem ersten Schichtbilddatensatz erzeugt werden. Beispielsweise kann eine volle Schnitt- bzw. Schichtbilddarstellung der Wirbelsäule in anterior-posterior-Sicht in einem zusammengesetzten Schichtbild dargestellt werden. Vorteilhaft kann eine anatomische Struktur, welche sich in einer Aufnahmeebene über mehrere Schichtbilder erstreckt, in einem zusammengesetzten Schichtbild angezeigt werden. Die Erfindung ermöglicht eine Diagnose basierend auf im Wesentlichen entsprechenden Ansichten einer Computertomographieaufnahme und dass, die Patientendosis reduziert wird.

Die Erfindung betrifft ferner eine Bilderzeugungseinheit zur Aufnahme von mindestens zwei um einen vorbestimmten Untersuchungswinkel versetzte Tomosyntheseaufnahmen eines Untersuchungsobjekts gemäß dem erfindungsgemäßen Verfahren aufweisend eine Aufnahmeeinheit, eine Ermittlungseinheit und eine Registriereinheit. Die Aufnahmeeinheit ist ausgebildet zum ersten Aufnehmen einer Mehrzahl von ersten Projektionsaufnahmen entlang der linearen Trajektorie, wobei die Röntgenquelle und der Röntgendetektor das Untersuchungsobjekt in einer ersten Aufnahmeebene aufnehmen und zum zweiten Aufnehmen einer Mehrzahl von zweiten Projektionsaufnahmen entlang der linearen Trajektorie, wobei die Röntgenquelle und der Röntgendetektor das Untersuchungsobjekt in einer von der ersten verschiedenen zweiten Aufnahmeebene aufnehmen, wobei die erste Aufnahmeebene und die zweite Aufnahmeebene den Untersuchungswinkel einschließen. Die Ermittlungseinheit ist ausgebildet zum Ermitteln von einem ersten Schichtbilddatensatz basierend auf den ersten Projektionsaufnahmen und von einem zweiten Schichtbilddatensatz basierend auf den zweiten Projektionsaufnahmen. Die Registriereinheit ist ausgebildet zum Registrieren von dem ersten Schichtbilddatensatz und dem zweiten Schichtbilddatensatz. Die Vorteile des erfindungsgemäßen Verfahrens können auf die Vorrichtung übertragen werden.

Ferner kann eine Rekonstruktionseinheit zum Rekonstruieren eines Tomosynthesedatensatzes vorgesehen sein, wobei die Tiefeninformation des Untersuchungsobjekts jeweils entlang von Röntgenstrahlen des Röntgenstrahlenbündels der Röntgenquelle ermittelt wird, so dass unterschiedliche Tiefenebenen im Objekt parallel zur Detektionsfläche eine unterschiedliche Abtastung aufweisen. Die Bilderzeugungseinheit kann ferner eine Darstellungseinheit zum Darstellen bzw. Anzeigen umfassen. Die Einheiten können zumindest teilweise von der Recheneinheit umfasst sein. Die Darstellungseinheit kann insbesondere als Anzeigeeinheit, beispielsweise in Form eines Bildschirms, ausgebildet sein.

Die Erfindung betrifft ferner ein medizinisches Röntgensystem aufweisend eine erfindungsgemäße Bilderzeugungseinheit zum Durchführen eines erfindungsgemäßen Verfahrens. Das medizinische Röntgensystem ist bevorzugt ein Radiographiesystem. Die Vorteile des erfindungsgemäßen Verfahrens können auf das medizinisches Röntgensystem übertragen werden.

Die Erfindung betrifft ferner ein Computerprogrammprodukt mit einem Computerprogramm, welches direkt in eine Speichereinrichtung einer Steuereinrichtung eines Röntgensystems ladbar ist, mit Programmabschnitten, um alle Schritte des erfindungsgemäßen Verfahrens auszuführen, wenn das Computerprogramm in der Steuereinrichtung des Röntgensystems ausgeführt wird.

Die Erfindung betrifft ferner ein computerlesbares Medium, auf welchem von einer Rechnereinheit einlesbare und ausführbare Programmabschnitte gespeichert sind, um alle Schritte des erfindungsgemäßen Verfahrens auszuführen, wenn die Programmabschnitte von der Recheneinheit ausgeführt werden. Die Recheneinheit kann bevorzugt von der Bilderzeugungseinheit bzw. einer Prozessoreinheit umfasst sein.

Nachfolgend werden Ausführungsbeispiele der Erfindung anhand von Zeichnungen näher erläutert. Hierbei zeigt:
FIG 1 eine schematische Darstellung des erfindungsgemäßen Röntgensystems in einer ersten Ausführungsform bei Aufnahme in der ersten Aufnahmeebene;
FIG 2 eine schematische Darstellung des erfindungsgemäßen Röntgensystems in einer ersten Ausführungsform bei Aufnahme in der zweiten Aufnahmeebene;
FIG 3 eine schematische Darstellung des erfindungsgemäßen Verfahrens;
FIG 4 eine schematische Darstellung des ersten Koordinatensystems relativ zum kartesischen Koordinatensystem;
FIG 5 eine schematische Darstellung einer Anzeige einer Abstandsbestimmung basierend auf dem ersten Schichtbilddatensatz und dem zweiten Schichtbilddatensatz;
FIG 6 eine schematische Darstellung der Anzeige des ersten Schichtbilds des ersten Schichtbilddatensatzes und des korrespondierenden zweiten Schichtbilds des zweiten Schichtbilddatensatzes; und
FIG 7 eine schematische Darstellung des erfindungsgemäßen Röntgensystems in einer zweiten Ausführungsform.

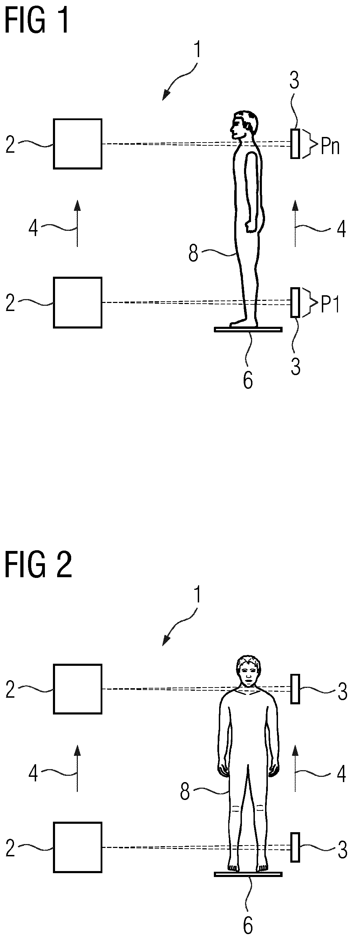

Die Fig. 1 zeigt eine beispielhafte Ausführung des erfindungsgemäßen Röntgensystems 1 in einer ersten Ausführungsform bei Aufnahme in der ersten Aufnahmeebene. Das Untersuchungsobjekt 8 ist zwischen einer Röntgenquelle 2 und einem Röntgendetektor 3 angeordnet. Das Untersuchungsobjekt 8 steht auf einer Standfläche 6. Die Röntgenquelle 2 und der Röntgendetektor 3 werden parallel gegenüberliegend entlang einer linearen Trajektorie 4 in die gleiche Bewegungsrichtung gleichzeitig bewegt.

Die Körperlängsachse des Untersuchungsobjekts 8 ist im Wesentlichen parallel zur linearen Trajektorie 4 ausgerichtet. Die lineare Trajektorie 4 kann beispielsweise entlang der Körperlängsachse bzw. parallel zur Körperlängsachse durch den Körpermittelpunkt verlaufen. Der Röntgendetektor 3 bewegt sich entlang der linearen Trajektorie 4 innerhalb einer Ebene, dabei spannt sich eine erste Aufnahmeebene auf.

Die Fig. 2 zeigt eine beispielhafte Ausführung des erfindungsgemäßen Röntgensystems 1 in einer ersten Ausführungsform bei Aufnahme in der zweiten Aufnahmeebene. Gegenüber der Fig. 1 ist der Patient um 90 Grad gedreht gegenüber der ersten Aufnahme angeordnet. Der Untersuchungswinkel beträgt damit 90 Grad. Bevorzugt hat sich das Röntgensystem 1 um 90 Grad verschieden gegenüber der ersten Aufnahme auf das Untersuchungsobjekt ausgerichtet. Der Röntgendetektor 3 bewegt sich entlang der linearen Trajektorie 4 innerhalb einer Ebene, dabei spannt sich eine zweite Aufnahmeebene auf. Die zweite Aufnahmeebene ist damit orthogonal zur ersten Aufnahmeebene ausgerichtet.

Alternativ kann sich der Röntgendetektor 3 und die Röntgenquelle 2 zur Aufnahme in der ersten und der zweiten Aufnahmeebene in gegenläufiger Richtung entlang der linearen Trajektorie 4 bewegen (nicht dargestellt). Alternativ kann sich nur die Röntgenquelle 2 zur Aufnahme in der ersten und der zweiten Aufnahmeebene entlang der linearen Trajektorie 4 bewegen, während der Röntgendetektor 3 fest an einer Position verbleibt (nicht dargestellt).

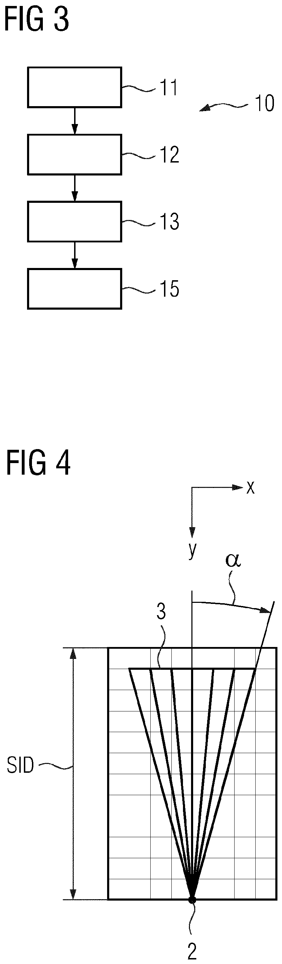

Die Fig. 3 zeigt eine beispielhafte Ausführung des erfindungsgemäßen Verfahrens 10. Das Verfahren 10 dient zur Aufnahme von mindestens zwei um einen vorbestimmten Untersuchungswinkel versetzte Tomosyntheseaufnahmen eines Untersuchungsobjekts. Das erfindungsgemäße Verfahren 10 weist die Schritte des ersten Aufnehmens 11, des zweiten Aufnehmens 12, des Ermittelns 13 und des Registrierens 15, bevorzugt in dieser Reihenfolge auf. Im Schritt des ersten Aufnehmens 11 wird eine Mehrzahl von ersten Projektionsaufnahmen entlang der linearen Trajektorie aufgenommen, wobei die Röntgenquelle und der Röntgendetektor das Untersuchungsobjekt in einer ersten Aufnahmeebene aufnehmen. Im Schritt des zweiten Aufnehmens 12 wird eine Mehrzahl von zweiten Projektionsaufnahmen entlang der linearen Trajektorie aufgenommen, wobei die Röntgenquelle und der Röntgendetektor das Untersuchungsobjekt in einer von der ersten verschiedenen zweiten Aufnahmeebene aufnehmen, wobei die erste Aufnahmeebene und die zweite Aufnahmeebene den Untersuchungswinkel einschließen. Im Schritt des zweiten Aufnehmens 12 können sich die Röntgenquelle und der Röntgendetektor in der gleichen Richtung oder in der entgegengesetzten Richtung im Vergleich zum ersten Aufnehmen 11 entlang der linearen Trajektorie bewegen. Im Schritt des Ermittelns 13 wird ein erster Schichtbilddatensatz basierend auf den ersten Projektionsaufnahmen und von einem zweiten Schichtbilddatensatz basierend auf den zweiten Projektionsaufnahmen ermittelt. Im Schritt des Registrierens 15 wird der erste Schichtbilddatensatz und der zweite Schichtbilddatensatz registriert.

Die Fig. 4 zeigt eine beispielhafte Ausführung des ersten Koordinatensystems relativ zum kartesischen Koordinatensystem. Das erste Koordinatensystem ist das aus der Aufnahmegeometrie resultierende native Koordinatensystem in der x-y-Ebene. Die Koordinaten eines Punktes zwischen der Röntgenquelle 2 und dem Röntgendetektor 3 werden nun durch die Koordinatentransformation (x,y,z) zu (α,y,z) mit α=sin(x/SID) beschrieben, wobei SID der Abstand zwischen Röntgenquelle 2 und Röntgendetektor 3 entlang der y-Richtung ist.

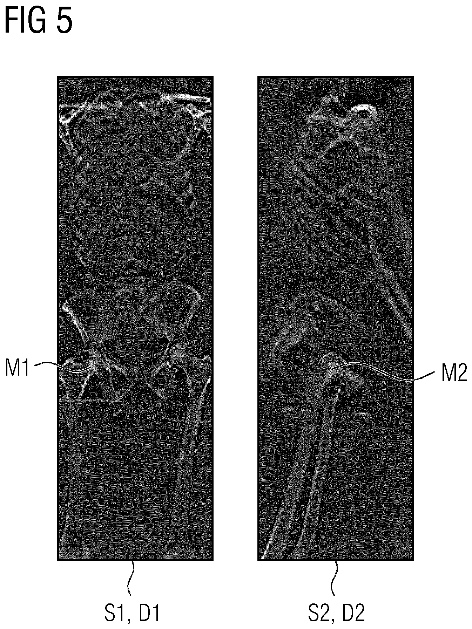

Die Fig. 5 zeigt eine Anzeige einer Abstandsbestimmung basierend auf dem ersten Schichtbilddatensatz und dem zweiten Schichtbilddatensatz. Basierend auf dem ersten Schichtbilddatensatz D1 und dem zweiten Schichtbilddatensatz D2 wird ein Abstand von anatomischen Merkmalen oder Landmarken bestimmt. Es werden insbesondere dreidimensionale Messungen von Knochenstellungen durchgeführt werden. Dazu werden anatomische Merkmale oder Landmarken im ersten Schichtbilddatensatz D1 und zweiten Schichtbilddatensatz D2 mit einer Markierung M1, M2 markiert. Das Markieren kann automatisch mittels einer automatischen Erkennung, beispielsweise basierend auf einer Segmentierung oder einer Bilderkennung, erfolgen. Alternativ oder zusätzlich kann das Markieren durch einen Benutzer erfolgen. Der Abstand zwischen den anatomischen Merkmalen bzw. Landmarken, wobei mindestens eine im ersten Schichtbilddatensatz D1 und mindestens eine andere im zweiten Schichtbilddatensatz D2 markiert ist, kann bestimmt werden, wobei jeweils der Pfadwinkel im ersten Schichtbilddatensatz D1 und im zweiten Schichtbilddatensatz D2 berücksichtigt wird. Die anatomischen Merkmale bzw. Landmarken sind insbesondere im ersten Schichtbild S1 des ersten Schichtbilddatensatzes D1 und im zweiten Schichtbild S2 des zweiten Schichtbilddatensatzes D2 verteilt und im jeweiligen Schichtbild mit der Markierung M1 bzw. M2 markiert.

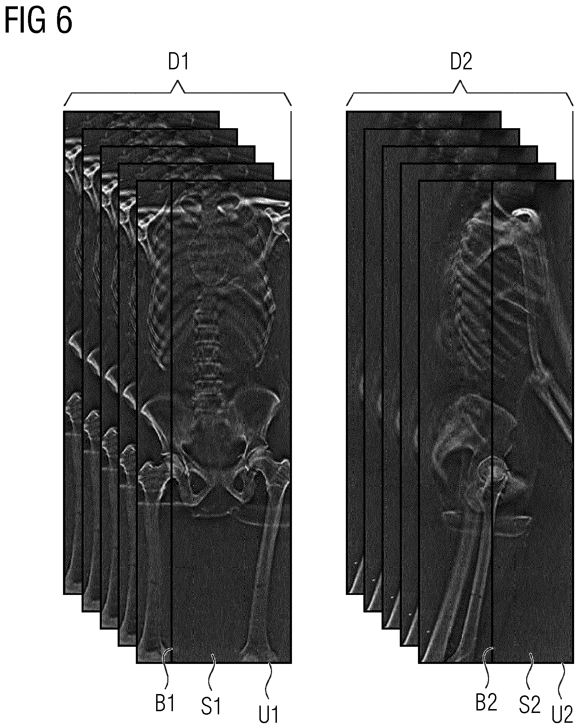

Die Fig. 6 zeigt eine beispielhafte Ausführung der erfindungsgemäßen Anzeige des ersten Schichtbilds S1 des ersten Schichtbilddatensatzes D1 und des korrespondierenden zweiten Schichtbilds S2 des zweiten Schichtbilddatensatzes D2. Mittels einer Benutzereingabe wird in einem ersten Schichtbild S1 des ersten Schichtbilddatensatzes D1 ein korrespondierendes zweites Schichtbild S2 im zweiten Schichtbilddatensatz D2 angezeigt. Der Benutzer kann mittels einer Eingabe, zum Beispiel durch Anklicken einer Position in einem Schichtbild S1,S2 des ersten Schichtbilddatensatzes D1 oder zweiten Schichtbilddatensatzes D2, die Anzeige des anderen Schichtbilddatensatzes D1,D2 beeinflussen, so dass beispielsweise die Position in beiden Schichtbilddatensätzen D1,D2 in dem die Position jeweils enthaltenen Schichtbild S1,S2 dargestellt wird. Bei der Bestimmung der Position im anderen Schichtbilddatensatz wird jeweils der Pfadwinkel im ersten Schichtbilddatensatz D1 und im zweiten Schichtbilddatensatz D2 berücksichtigt. Es wird eine Transformation einer Position von einem Schichtbilddatensatz in den anderen Schichtbilddatensatz durchgeführt.

Mittels der Benutzereingabe wird ein Schieberegler B1 in einem ersten Schichtbild S1 des ersten Schichtbilddatensatzes D2 bewegt, so dass das der Tiefe des momentan angezeigten Schiebereglers B1 entsprechende zweite Schichtbild S2 des zweiten Schichtbilddatensatzes D2 angezeigt wird. Durch Betätigen bzw. Verschieben des Schiebereglers B1 in einem ersten Schichtbild S1 des ersten Schichtbilddatensatzes D1 wird das entsprechend anzuzeigende zweite Schichtbild S2 des zweiten Schichtbilddatensatzes D2 ausgewählt. Die Anzeige des ersten Schichtbilddatensatzes D1 und zweiten Schichtbilddatensatzes D2 wird damit synchronisiert. Wird der vertikaler Balken B1 im ersten Schichtbild S1 an einem x- bzw. α-Wert angezeigt, so wird als zweites Schichtbild S2 dasjenige Schichtbild des zweiten Schichtbilddatensatzes D2 angezeigt, wobei die Tiefe bzw. der y-Wert des zweiten Schichtbildes S2 dem x- bzw. α-Wert im ersten Schichtbild S1 im Wesentlichen entspricht. Das erste Schichtbild S1 wird in Form einer Umrandung U1 entlang der Außenkanten des ersten Schichtbilds S1 markiert. Die Umrandung U1 und der Schieberegler B2 im zweiten Schichtbild S2 sind farblich identisch kodiert. Die Tiefe bzw. der y-Wert des ersten Schichtbildes S1 kann entsprechend mittels eines Schiebereglers B2 an einem entsprechenden x- bzw. α-Wert im zweiten Schichtbild S2 eingeblendet bzw. angezeigt werden. Das zweite Schichtbild S2 weist eine Umrandung U2 entlang der Außenkanten des zweiten Schichtbilds S2 auf. Die Umrandung U2 und der Schieberegler B1 im ersten Schichtbild S1 können farblich identisch kodiert werden, wobei sich die Farbe von der Kodierung des ersten Schichtbilds S1 und des Schiebereglers B2 im zweiten Schichtbild S2 unterscheidet. Der Schieberegler B1, B2 wird in Form eines Balkens entlang der vertikalen z-Achse in einem Schichtbild des ersten Schichtbilddatensatzes D1 oder zweiten Schichtbilddatensatzes D2 eingeblendet.

Der zweite Schichtbilddatensatz D2 wird mittels Bewegens des Schiebereglers B1 bzw. Balkens, der im ersten Schichtbilddatensatz D1 eingeblendet bzw. überlagert angezeigt wird, durchscrollt. Gleiches ist auch anders herum möglich, also dass der erste Schichtbilddatensatz D1 mittels Bewegens des Schiebereglers B2 bzw. Balkens, der im zweiten Schichtbilddatensatz D2 eingeblendet bzw. überlagert angezeigt wird, durchscrollt werden kann.

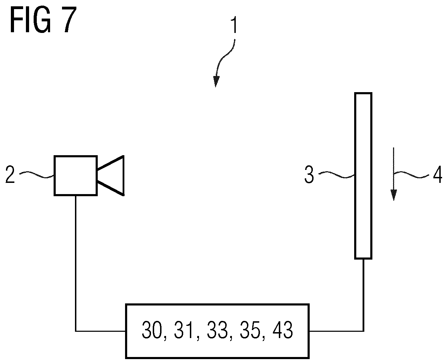

Die Fig. 7 zeigt eine beispielhafte Ausführung der erfindungsgemäßen Röntgensystems 1 in einer zweiten Ausführungsform. Die Röntgenquelle 2 und der Röntgendetektor 3 sind über eine Bilderzeugungseinheit 30 miteinander verbunden. Die Bilderzeugungseinheit kann eine Recheneinheit und/oder Steuereinheit bzw. Steuereinrichtung sein bzw. von dieser umfasst sein. Die Bilderzeugungseinheit 30 umfasst die Aufnahmeeinheit 31, die Ermittlungseinheit 33 und die Registriereinheit 35. Ferner kann die Bilderzeugungseinheit eine Darstellungseinheit 43, welche beispielsweise als Bildschirm ausgebildet ist, aufweisen.

Obwohl die Erfindung im Detail durch das bevorzugte Ausführungsbeispiel näher illustriert wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren (10) zur Aufnahme von mindestens zwei um einen vorbestimmten Untersuchungswinkel versetzte Tomosyntheseaufnahmen eines Untersuchungsobjekts (8), welches zwischen einer Röntgenquelle (2) und einem Röntgendetektor (3) angeordnet ist und wobei die Röntgenquelle und/oder der Röntgendetektor in gegenüberliegenden parallelen Ebenen entlang einer linearen Trajektorie (4) bewegt werden und wobei die Körperlängsachse des Untersuchungsobjekts oder eine Längsachse der Untersuchungsregion des Untersuchungsobjekts im Wesentlichen parallel zur linearen Trajektorie ausgerichtet ist, aufweisend die Schritte:
- erstes Aufnehmen (11) einer Mehrzahl von ersten Projektionsaufnahmen entlang der linearen Trajektorie, wobei die Röntgenquelle und der Röntgendetektor das Untersuchungsobjekt in einer ersten Aufnahmeebene aufnehmen,
- zweites Aufnehmen (12) einer Mehrzahl von zweiten Projektionsaufnahmen entlang der linearen Trajektorie, wobei die Röntgenquelle und der Röntgendetektor das Untersuchungsobjekt in einer von der ersten verschiedenen zweiten Aufnahmeebene aufnehmen, wobei die erste Aufnahmeebene und die zweite Aufnahmeebene den Untersuchungswinkel einschließen,
- Ermitteln (13) von einem ersten Schichtbilddatensatz (D1) basierend auf den ersten Projektionsaufnahmen und von einem zweiten Schichtbilddatensatz (D2) basierend auf den zweiten Projektionsaufnahmen, wobei der erste Schichtbilddatendatz und der zweite Schichtbilddatendatz im Wesentlichen entsprechende Ansichten einer Computertomographieaufnahme bereitstellen und die Patientendosis gegenüber der Computertomographieaufnahme reduziert ist, und
- Registrieren (15) von dem ersten Schichtbilddatensatz (D1) und dem zweiten Schichtbilddatensatz (D2) .

2. Verfahren nach Anspruch 1, wobei beim Ermitteln des ersten Schichtbilddatensatzes und des zweiten Schichtbilddatendatensatzes die Tiefeninformation jeweils entlang eines Pfades mit einem Pfadwinkel (α) gegenüber einer kartesischen Raumrichtung ermittelt wird.

3. Verfahren nach Anspruch 2, wobei im Schritt des Registrierens jeweils der Pfadwinkel im ersten Schichtbilddatensatz und im zweiten Schichtbilddatensatz berücksichtigt wird.

4. Verfahren nach einem der vorangehenden Ansprüche, wobei der Untersuchungswinkel untersuchungsabhängig ist.

5. Verfahren nach einem der vorangehenden Ansprüche, wobei der Untersuchungswinkel im Wesentlichen 90 Grad beträgt.

6. Verfahren nach einem der vorangehenden Ansprüche, wobei basierend auf dem ersten Schichtbilddatensatz und dem zweiten Schichtbilddatensatz ein Abstand von anatomischen Merkmalen oder Landmarken bestimmt wird.

7. Verfahren nach einem der vorangehenden Ansprüche, wobei der erste Schichtbilddatensatz und der zweite Schichtbilddatensatz gemeinsam angezeigt werden.

8. Verfahren nach Anspruch 7, wobei mittels einer Benutzereingabe in einem ersten Schichtbild (S1) des ersten Schichtbilddatensatzes ein korrespondierendes zweites Schichtbild (S2) im zweiten Schichtbilddatensatz angezeigt wird.

9. Verfahren nach Anspruch 8, wobei mittels der Benutzereingabe ein Schieberegler (B1) in einem ersten Schichtbild des ersten Schichtbilddatensatz bewegt wird, so dass das der Tiefe des momentan angezeigten Schiebereglers entsprechende zweite Schichtbild des zweiten Schichtbilddatensatzes angezeigt wird.

10. Verfahren nach einem der voranstehenden Ansprüche, wobei der Verlauf einer sich erstreckenden anatomischen Struktur in einem ersten Schichtbild des ersten Schichtbilddatensatzes markiert wird und ein zusammengesetztes Schichtbild basierend auf dem markierten Verlauf und basierend auf dem zweiten Schichtbilddatensatz ermittelt wird.

11. Bilderzeugungseinheit (30) zur Aufnahme von mindestens zwei um einen vorbestimmten Untersuchungswinkel versetzte Tomosyntheseaufnahmen eines auf einer Standfläche (6) stehenden Untersuchungsobjekts nach einem der vorangehenden Ansprüche aufweisend:
- Aufnahmeeinheit (31) zum erstes Aufnehmen (11) einer Mehrzahl von ersten Projektionsaufnahmen entlang der linearen Trajektorie, wobei die Röntgenquelle und der Röntgendetektor das Untersuchungsobjekt in einer ersten Aufnahmeebene aufnehmen und zum zweiten Aufnehmen (12) einer Mehrzahl von zweiten Projektionsaufnahmen entlang der linearen Trajektorie, wobei die Röntgenquelle und der Röntgendetektor das Untersuchungsobjekt in einer von der ersten verschiedenen zweiten Aufnahmeebene aufnehmen, wobei die erste Aufnahmeebene und die zweite Aufnahmeebene den Untersuchungswinkel einschließen,
- Ermittlungseinheit (35) zum Ermitteln (13) von einem ersten Schichtbilddatensatz basierend auf den ersten Projektionsaufnahmen und von einem zweiten Schichtbilddatensatz basierend auf den zweiten Projektionsaufnahmen, wobei der erste Schichtbilddatendatz und der zweite Schichtbilddatendatz im Wesentlichen entsprechende Ansichten einer Computertomographieaufnahme bereitstellen und die Patientendosis gegenüber der Computertomographieaufnahme reduziert ist,
- Registriereinheit (37) zum Registrieren (15) von dem ersten Schichtbilddatensatz und dem zweiten Schichtbilddatensatz.

12. Medizinisches Röntgensystem (1) aufweisend eine Bilderzeugungseinheit nach Anspruch 11.

13. Computerprogrammprodukt mit einem Computerprogramm, welches direkt in eine Speichereinrichtung einer Steuereinrichtung eines Röntgensystems (1) ladbar ist, mit Programmabschnitten, um alle Schritte eines Verfahrens nach einem der Ansprüche 1 bis 10 auszuführen, wenn das Computerprogramm in der Steuereinrichtung des Röntgensystems (1) ausgeführt wird.

14. Computerlesbares Medium, auf welchem von einer Rechnereinheit einlesbare und ausführbare Programmabschnitte gespeichert sind, um alle Schritte eines Verfahrens nach einem der Ansprüche 1 bis 10 auszuführen, wenn die Programmabschnitte von der Recheneinheit ausgeführt werden.

## Claims

1. Method (10) for capturing at least two tomosynthesis images of an object undergoing examination (8) that are offset by a predetermined angle of examination, which object undergoing examination (8) is arranged between an X-ray source (2) and an X-ray detector (3), and wherein the X-ray source and/or the X-ray detector are moved in opposing parallel planes along a linear trajectory (4) and wherein the longitudinal axis of the body of the object undergoing examination or a longitudinal axis of the region of the object undergoing examination that is being examined is oriented substantially parallel to the linear trajectory, having the following steps:
- first capture (11) of a plurality of first projection images along the linear trajectory, wherein the X-ray source and the X-ray detector capture the object undergoing examination in a first plane of capture,
- second capture (12) of a plurality of second projection images along the linear trajectory, wherein the X-ray source and the X-ray detector capture the object undergoing examination in a second capture plane that is different from the first, wherein the first capture plane and the second capture plane form the angle of examination,
- determination (13) of a first slice image dataset (D1) on the basis of the first projection images and of a second slice image dataset (D2) on the basis of the second projection images, wherein the first slice image dataset and the second slice image dataset provide substantially corresponding views of a capture using computed tomography and the patient dose is reduced compared to capture using computed tomography, and
- registration (15) of the first slice image dataset (D1) and the second slice image dataset (D2).

2. Method according to claim 1, wherein, when the first slice image dataset and the second slice image dataset are determined, the depth information is determined in each case along a path having a path angle (α) in relation to a Cartesian direction in space.

3. Method according to claim 2, wherein in the registration step the path angle is taken into account in each case in the first slice image dataset and the second slice image dataset.

4. Method according to one of the preceding claims, wherein the angle of examination depends on the examination.

5. Method according to one of the preceding claims, wherein the angle of examination is substantially 90 degrees.

6. Method according to one of the preceding claims, wherein a spacing between anatomical features or landmarks is determined on the basis of the first slice image dataset and the second slice image dataset.

7. Method according to one of the preceding claims, wherein the first slice image dataset and the second slice image dataset are displayed together.

8. Method according to claim 7, wherein by means of a user input in a first slice image (S1) of the first slice image dataset a corresponding second slice image (S2) in the second slice image dataset is displayed.

9. Method according to claim 8, wherein the user input moves a slide control (B1) in a first slice image of the first slice image dataset, with the result that the second slice image of the second slice image dataset, corresponding to the depth of the currently displayed slide control, is displayed.

10. Method according to one of the preceding claims, wherein the course taken by an extended anatomical structure in a first slice image of the first slice image dataset is marked, and an assembled slice image based on the marked course and on the second slice image dataset is determined.

11. Image generating unit (30) for capturing at least two tomosynthesis images, which are offset by a predetermined angle of examination, of an object undergoing examination standing on a base surface (6) according to one of the preceding claims, having:
- a receiving unit (31) for the first capture (11) of a plurality of first projection images along the linear trajectory, wherein the X-ray source and the X-ray detector capture the object undergoing examination in a first capture plane, and for the second capture (12) of a plurality of second projection images along the linear trajectory, wherein the X-ray source and the X-ray detector capture the object undergoing examination in a second capture plane that is different from the first, wherein the first capture plane and the second capture plane form the angle of examination,
- a determining unit (35) for the determination (13) of a first slice image dataset on the basis of the first projection images and a second slice image dataset on the basis of the second projection images, wherein the first slice image dataset and the second slice image dataset provide substantially corresponding views of a capture using computed tomography and the patient dose is reduced compared to capture using computed tomography,
- a registering unit (37) for the registration (15) of the first slice image dataset and the second slice image dataset.

12. Medical X-ray system (1) having an image generating unit according to claim 11.

13. Computer program product having a computer program that can be loaded directly into a memory facility of a control facility of an X-ray system (1), having program sections in order to perform all the steps of a method according to one of claims 1 to 10 when the computer program is executed in the control facility of the X-ray system (1).

14. A computer-readable medium on which program sections that can be read and executed by a computer unit are stored in order to perform all the steps of a method according to one of claims 1 to 10 when the program sections are executed by the computing unit.

## Revendications

1. Procédé (10) d'enregistrement d'au moins deux enregistrements de tomosynthèse, décalés d'un angle d'examen déterminé à l'avance, d'un objet (8) à examiner, qui est disposé entre une source (2) de rayons X et un détecteur (3) de rayons X et dans lequel on déplace la source de rayons X et/ou le détecteur de rayons X dans des plans parallèles opposés le long d'une trajectoire (4) linéaire et dans lequel l'axe longitudinal du corps de l'objet à examiner ou un axe longitudinal d'une région à examiner de l'objet à examiner est dirigé sensiblement parallèlement à la trajectoire linéaire, comportant les stades :
- premier enregistrement (11) d'une pluralité de premiers enregistrements de projection le long de la trajectoire linéaire, dans lequel la source de rayons X et le détecteur de rayons X enregistrent l'objet à examiner dans un premier plan d'enregistrement,
- deuxième enregistrement (12) d'une pluralité de deuxièmes enregistrements de projection le long de la trajectoire linéaire, dans lequel la source de rayons X et le détecteur de rayons X enregistrent l'objet à examiner dans un deuxième plan d'enregistrement différent du premier, dans lequel le premier plan d'enregistrement et le deuxième plan d'enregistrement incluent l'angle d'examen,
- détermination (13) d'un premier ensemble (D1) de données d'image de couche reposant sur les premiers enregistrements de projection et d'un deuxième ensemble (D2) de données d'image de couche reposant sur les deuxièmes enregistrements de projection, dans lequel le premier ensemble de données d'image de couche et le deuxième ensemble de données d'image de couche donnent des vues sensiblement correspondantes d'un enregistrement de tomodensitométrie assistée par ordinateur et la dose au patient est réduite par rapport à l'enregistrement de tomodensitométrie assistée par ordinateur et,
- enregistrement (15) du premier ensemble (D1) de données d'image de couche et du deuxième ensemble (D2) de données d'image de couche.

2. Procédé suivant la revendication 1, dans lequel, lors de la détermination du premier ensemble de données d'image de couche et du deuxième ensemble de données d'image de couche, on détermine l'information de profondeur respectivement le long d'un chemin faisant un angle (a) de chemin par rapport à une direction cartésienne de l'espace.

3. Procédé suivant la revendication 2, dans lequel, dans le stade de l'enregistrement, on tient compte respectivement de l'angle du chemin dans le premier ensemble de données d'image de couche et dans le deuxième ensemble de données d'image de couche.

4. Procédé suivant l'une des revendications précédentes, dans lequel l'angle d'examen dépend de l'examen.

5. Procédé suivant l'une des revendications précédentes, dans lequel l'angle d'examen est sensiblement de 90 degrés.

6. Procédé suivant l'une des revendications précédentes, dans lequel on détermine une distance de caractéristiques anatomiques ou de jalons sur la base du premier ensemble de données d'image de couche et du deuxième ensemble de données d'image de couche.

7. Procédé suivant l'une des revendications précédentes, dans lequel on affiche conjointement le premier ensemble de données d'image de couche et le deuxième ensemble de données d'image de couche.

8. Procédé suivant la revendication 7, dans lequel, au moyen d'une entrée d'utilisateur, on affiche, dans une première image (S1) de couche du premier ensemble de données d'image de couche, une deuxième image (S2) de couche correspondante du deuxième ensemble de données d'image de couche.

9. Procédé suivant la revendication 8, dans lequel, au moyen de l'entrée d'utilisateur, on déplace un régleur (B1) coulissant dans une première image de couche du premier ensemble de données d'image de couche, de manière à afficher la deuxième image de couche, correspondant à la profondeur du régleur coulissant affiché instantanément, du deuxième ensemble de données d'image de couche.

10. Procédé suivant l'une des revendications précédentes, dans lequel on repère dans une première image de couche du premier ensemble de données d'image de couche le tracé d'une structure anatomique, qui a de l'étendue, et on détermine une image de couche composée sur la base du tracé repéré et sur la base du deuxième ensemble de données d'image de couche.

11. Unité (30) de production d'image pour l'enregistrement d'au moins deux enregistrements de tomosynthèse, décalés d'un angle d'examen déterminé à l'avance, d'un objet à examiner debout sur une surface (6) de base suivant l'une des revendications précédentes comportant :
- une unité (31) d'enregistrement pour le premier enregistrement (11) d'une pluralité de premiers enregistrements de projection le long de la trajectoire linéaire, dans laquelle la source de rayons X et le détecteur de rayons X enregistrent l'objet à examiner dans un premier plan d'enregistrement et pour le deuxième enregistrement (12) une pluralité de deuxièmes enregistrements de projection le long de la trajectoire linéaire, dans laquelle la source de rayons X et le détecteur de rayons X enregistrent l'objet à examiner dans un deuxième plan d'enregistrement différent du premier, dans laquelle le premier plan d'enregistrement et le deuxième plan d'enregistrement incluent l'angle d'examen,
- une unité (35) de détermination pour la détermination (13) d'un premier ensemble de données d'image de couche reposant sur les premiers enregistrements de projection et d'un deuxième ensemble de données d'image de couche reposant sur les deuxièmes enregistrements de projection, dans laquelle le premier ensemble de données d'image de couche et le deuxième ensemble de données d'image de couche donnent des vues sensiblement correspondantes d'un enregistrement de tomodensitométrie assisté par ordinateur et la dose au patient est réduite par rapport à l'enregistrement de tomodensitométrie assistée par ordinateur,
- une unité (37) d'enregistrement pour l'enregistrement (15) du premier ensemble de données d'image de couche et du deuxième ensemble de données d'image de couche.

12. Système (1) de rayons X médical comportant une unité de production d'image suivant la revendication 11.

13. Produit de programme d'ordinateur ayant un programme d'ordinateur, qui peut être chargé directement dans un dispositif de mémoire d'un dispositif de commande d'un système (1) à rayons X, comprenant des parties de programme pour effectuer tous les stades d'un procédé suivant l'une des revendications 1 à 10, lorsque le programme d'ordinateur est réalisé dans le dispositif de commande du système (1) à rayons X.

14. Support, déchiffrable par ordinateur, sur lequel sont mises en mémoire des parties de programme pouvant être lues et réalisées par une unité informatique afin d'effectuer tout le stade d'un procédé suivant l'une des revendications 1 à 10, lorsque les parties de programme sont réalisées par l'unité informatique.
